(19) Europäisches Patentamt
European Patent Office
Office européen des brevets

(11) EP 1 902 737 A1

(12) **EUROPEAN PATENT APPLICATION**
published in accordance with Art. 153(4) EPC

(43) Date of publication:
**26.03.2008 Bulletin 2008/13**

(21) Application number: **06757015.0**

(22) Date of filing: **06.06.2006**

(51) Int Cl.:
***A61L 27/00*** *(2006.01)* ***C12M 3/00*** *(2006.01)*
***G02C 7/04*** *(2006.01)* ***C08F 30/08*** *(2006.01)*

(86) International application number:
**PCT/JP2006/311275**

(87) International publication number:
**WO 2007/007489 (18.01.2007 Gazette 2007/03)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **08.07.2005 JP 2005200920**

(71) Applicant: **Menicon Co., Ltd.**
**Nagoya-shi, Aichi 460-0006 (JP)**

(72) Inventors:
- **UEHARA, Katsuhiro,**
**c/o MENICON CO., LTD.**
**Kasugai-shi,**
**Aichi 4870032 (JP)**
- **KUBOTA, Tohru,**
**c/o MENICON CO., LTD.**
**Kasugai-shi,**
**Aichi 4870032 (JP)**
- **SUGIE, Toshimasa,**
**c/o MENICON CO., LTD.**
**Kasugai-shi,**
**Aichi 4870032 (JP)**
- **FUJIWARA, Akihito,**
**MENICON CO., LTD.**
**Kasugai-shi,**
**Aichi4870032 (JP)**
- **YOKOYAMA, Yasuhiro,**
**c/o MENICON CO., LTD.**
**Kasugai-shi,**
**Aichi 4870032 (JP)**

(74) Representative: **TBK-Patent**
**Bavariaring 4-6**
**80336 München (DE)**

(54) **OPHTHALMIC LENS, CELL OR ORGAN CULTURE SUBSTRATE, CONTAINER FOR BIOLOGICAL MATERIAL AND TRANSPARENT GEL PRODUCED BY POLYMERIZATION OF CYCLIC SILOXANE COMPOUND AND PROCESS FOR PRODUCTION THEREOF**

(57) The present invention is to provide an ophthalmic lens, a culture substrate material for cells or organs, a container for living things and a transparent gel wherein wettability and tackiness are excellent and no surface treatment is required. The ophthalmic lens, the culture substrate material for cells and organs and the transparent gel is obtained by polymerizing a cyclic siloxane compound shown by the following formula (A)

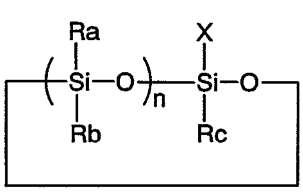

(A)

(wherein Ra or Rb is a hydrogen atom or a monovalent hydrocarbon group which may be substituted by a fluorine atom, and they are same or different with each other, Rc is a C1 to C6 alkyl group or a phenyl group, X is an organic group containing an unsaturated aliphatic bond, and n is an integer of 1 to 10).

EP 1 902 737 A1

## Description

TECHNICAL FIELD

The present invention relates to an ophthalmic lens, a culture substrate material for cells or organs, a container for a living thing and a transparent gel, which are obtained by polymerizing a cyclic siloxane compound.

BACKGROUND ART

A conventional polymer obtained from siloxane monomer has been highly tacky and sticky and thus difficult to handle. Further, the resulting polymer has been necessary for a surface treatment, and this surface treatment has been accompanied with such a problem that the processes are complicated and the effect is lowered after using for a long period.
For instance, in Japanese Patent Publication No. 1-256537A, a contact lens comprising a cyclic or straight chained siloxane, which is wettable, rigid, gas-permeable and substantially non-expansible is disclosed. However, as the cyclic siloxane compound contains no cross-linkable unsaturated group, it is difficult to produce a transparent gel, and even when it can be produced, its transparency and mechanical strength are not practically applicable.
Further, in Japanese Patent Publication No. 7-149902A, a cyclic siloxane compound is disclosed, but this is for using to a silicone type releasing paper for giving hardening property and no consideration is made at all to application to a substrate material for an ophthalmic lens and other living body-related substances and to a container for living things.

DISCLOSURE OF INVENTION

The present invention provides an ophthalmic lens which is required for no surface treatment and excellent in tackiness, a culture substrate material for cells or organs, a container for living things and a transparent gel.
Namely, the present invention relates to an ophthalmic lens obtained by polymerizing a cyclic siloxane compound shown by the following formula (A).

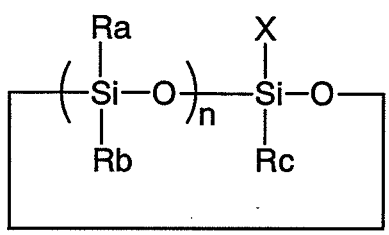

(A)

(wherein Ra or Rb is a hydrogen atom or a monovalent hydrocarbon group which may be substituted by a fluorine atom, and they are same or different with each other, Rc is a C 1 to C6 alkyl group or a phenyl group, X is an organic group containing an unsaturated aliphatic bond, and n is an integer of 1 to 10).
The cyclic siloxane compound is preferably polymerized together with a hydrophilic monomer.
Further, the present invention relates to a culture substrate material for cells or organs, which contains the cyclic siloxane compound shown by the following formula (A).

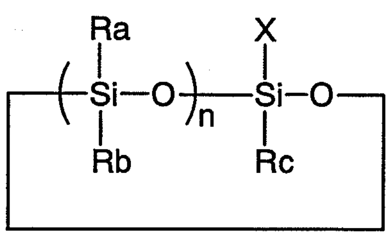

(A)

(wherein Ra or Rb is a hydrogen atom or a monovalent hydrocarbon group which may be substituted by a fluorine atom, and they are same or different with each other, Rc is a C 1 to C6 alkyl group or a phenyl group, X is an organic group containing an unsaturated aliphatic bond, and n is an integer of 1 to 10).

It is preferable to polymerize a hydrophilic monomer together with the cyclic siloxane compound.

The present invention also relates to a container for living things, which is obtained by polymerizing the cyclic siloxane compound shown by the following formula (A).

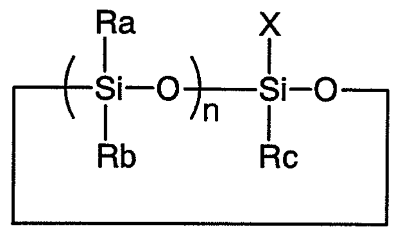

(A)

(wherein Ra or Rb is a hydrogen atom or a monovalent hydrocarbon group which may be substituted by a fluorine atom, and they are same or different with each other, Rc is a C 1 to C6 alkyl group or a phenyl group, X is an organic group containing an unsaturated aliphatic bond, and n is an integer of 1 to 10).

The present invention still further relates to a transparent gel obtained by polymerizing the cyclic siloxane compound shown by the following formula (A), a hydrophilic monomer and a thermosetting polyimide silicone resin soluble in organic solvents which contains a structural units shown by the following formula (B-1) and one shown by the following formula (B-2).

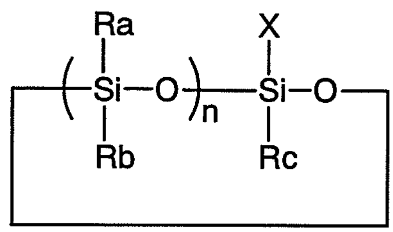

(A)

(wherein Ra or Rb is a hydrogen atom or a monovalent hydrocarbon group which may be substituted by a fluorine atom, and they are same or different with each other, Rc is a C 1 to C6 alkyl group or a phenyl group, X is an organic group containing an unsaturated aliphatic bond, and n is an integer of 1 to 10).

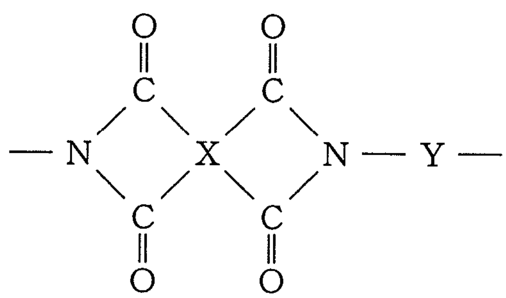

(B-1)

[wherein X is a tetravalent organic group having 4 or more carbon atoms, providing that plural of -CO- groups are not bound to one carbon atom in X, and Y is a diamine residue shown by the general formula (1) or (2)

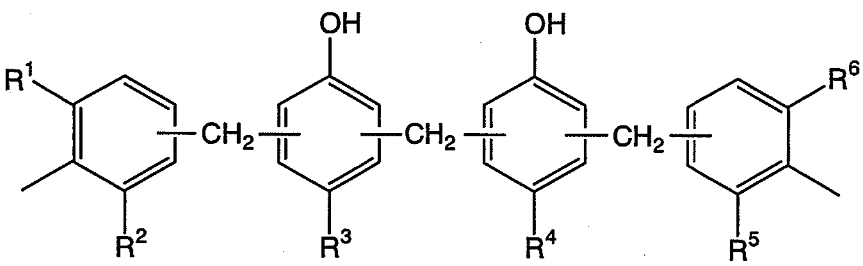

(1)

(wherein each of $R^1$ to $R^6$ is, same or different, a hydrogen atom or a C1 to C6 alkyl group).

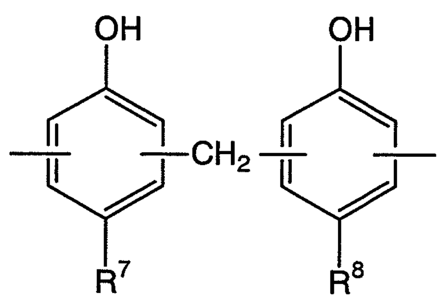

(2)

(wherein $R^7$ and $R^8$ are, same or different, a hydrogen atom or a C 1 to C6 alkyl group)].

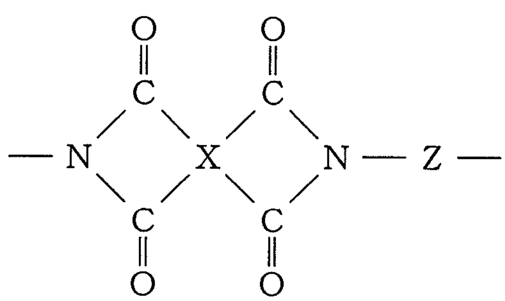

(B-2)

[wherein X is a tetravalent organic group having 4 or more carbon atoms, providing that plural of -CO- groups are not bound to one carbon atom in X, and Z is a diamine residue shown by the general formula (3)

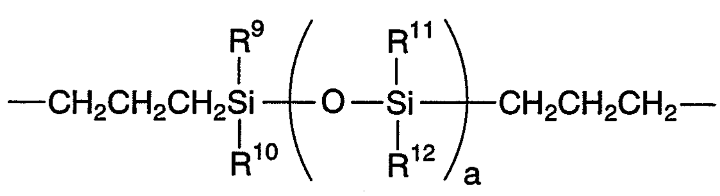

(3)

(wherein $R^9$ to $R^{12}$ are, same or different, a substituted or unsubstituted monovalent hydrocarbon group of C1 to C8, and a is an integer of 1 to 100)].

Further, the present invention relates to a method for producing a transparent gel, comprising dissolving a polyimide silicone resin containing structural units shown by the formula (B-1) and (B-2) into a mixture of a cyclic siloxane compound shown by the formula (A) and a hydrophilic monomer, followed by polymerization.

BEST MODE FOR CARRYING OUT THE INVENTION

The ophthalmic lens, a culture substrate material for cells and organs and a container for living things of the present invention is obtained by polymerizing a cyclic siloxane compound shown by the following formula (A).

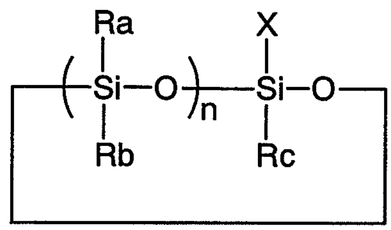

(A)

(wherein Ra or Rb is a hydrogen atom or a monovalent hydrocarbon group which may be substituted by a fluorine atom, and they are same or different with each other, Rc is a C 1 to C6 alkyl group or a phenyl group, X is an organic group containing an unsaturated aliphatic bond, and n is an integer of 1 to 10).

Ra or Rb is a hydrogen atom or a monovalent hydrocarbon group which may be substituted by a fluorine atom, and they are same or different with each other. Preferably, Ra and Rb are a C1 1 to C6 alkyl group and still preferably Ra is a C 1 to C6 alkyl group substituted by a fluorine atom and Rb is a C1 to C6 alkyl group. The C1 to C6 alkyl group in Ra and Rb is exemplified by a methyl group, an ethyl group, a propyl group, an isopropyl group, a butyl group, an isobutyl group, a tert-butyl group, a pentyl group, an isopentyl group, a hexyl group, etc. Among the above, one wherein Ra is $-CH_3$ or $-CH_2CH_2CF_3$ and Rb is $-CH_3$ is still preferable. In a case where Ra or Rb is a monovalent hydrocarbon group such as an alkyl group of 7 or more carbon atoms, the obtained lens tends to be stuck with a lipid or tends to become lowered flexible. In a case where Ra is an alkyl group substituted by a fluorine atom, Ra has preferably 3 to 6 carbon atoms, and when the carbon atom is less than 3, the compound containing an alkyl group substituted by a fluorine atom tends to be difficult to produce.

The X in the above formula (A) contains an unsaturated aliphatic group which is exemplified by the following

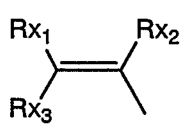

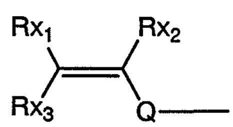

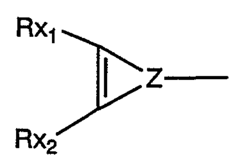

(wherein $Rx_1$, $Rx_2$ and $Rx_3$ are a hydrogen atom, a C 1 to C6 alkyl group or a phenyl group, which may be same or different, Q is a divalent organic group, and Z is a trivalent organic group).

The Q in the above formula (A) is preferably (i) a C1 to C8 straight chained or branched alkylene group or (ii) a C1 to C8 straight chained or branched alkylene group whose methylene unit is substituted by one or more members selected from the group consisting of -O-, -CO-, -COO-, -OCO-, -OCOO-, $-C_6H_4-$, $-OC_6H_4-$ and -S-, including specifically a methylene group, an ethylene group, a propylene group, a butylene group, a pentylene group, a hexylene group, an isobutylene group, an isopentylene group, an ethylhexylene group, etc.

The Z in the formula (A) is preferably (iii) a C1to C20 trivalent hydrocarbon group forming a cyclic structure through an unsaturated aliphatic bond as the structural unit to which $Rx_1$ and $Rx_2$ are bound or (iv) a C2 to C20 trivalent hydrocarbon group whose methylene unit is substituted by one or more members selected from the group consisting of -O-, -CO-, -COO-, -OCO-, -OCOO-, $-C_6H_4-$, $-OC_6H_4-$ and -S-.

Among the above, as the X in the formula A, the following is preferable from viewpoints of low cost in an industrial scale and availability in a large amount

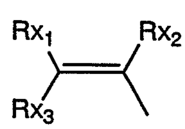

wherein $Rx_1$, $Rx_2$ and $Rx_3$ are a hydrogen atom or a methyl group, which may be same or different,

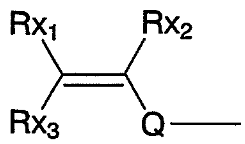

(4)

in the formula (4), $Rx_1$, $Rx_2$ and $Rx_3$ are a hydrogen atom or a methyl group, which may be same or different, Q is a substituent shown by the formula (5) and m is an integer of 3.

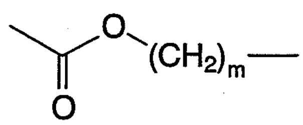

(5)

or

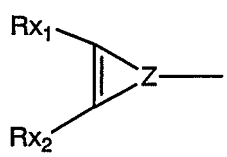

wherein $Rx_1$ and $Rx_2$ are a hydrogen atom or a methyl group, which may be same or different, and Z is

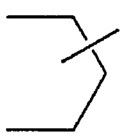

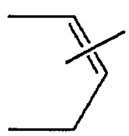

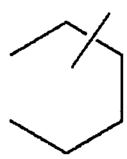

or

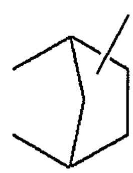

.

Specific examples of the above substituents are as the followings, but the substituent X in the present invention is not limited thereto.

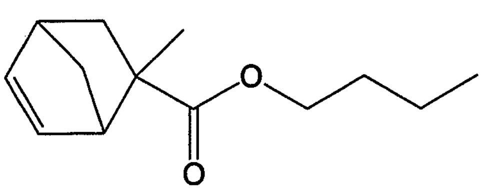

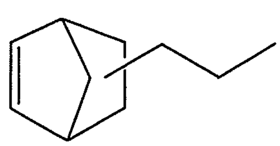

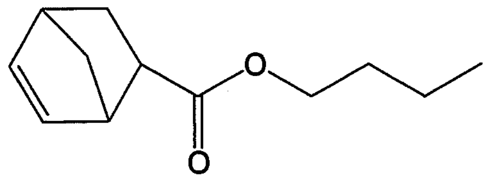

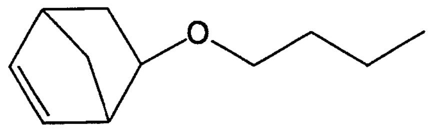

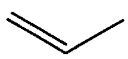

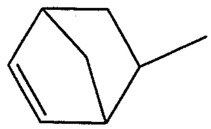

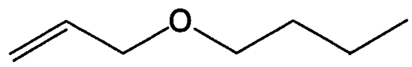

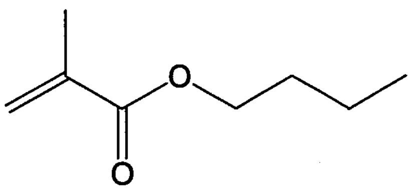

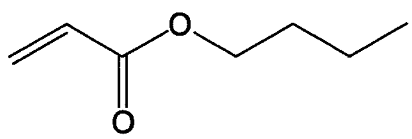

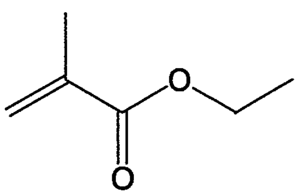

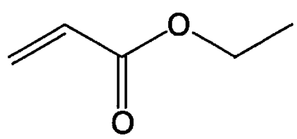

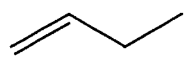

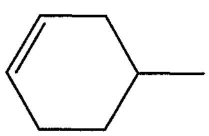

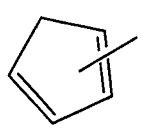

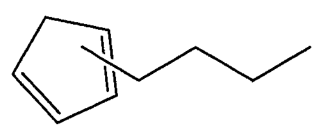

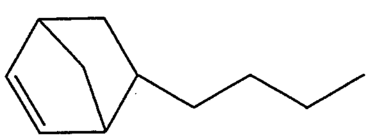

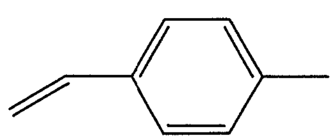

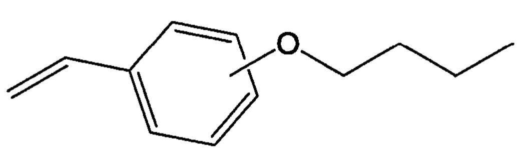

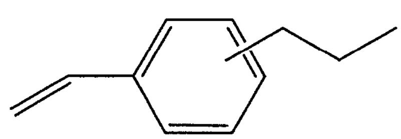

The Rc in the formula (A) is a C1 to C6 alkyl group or a phenyl group, including specifically the alkyl group mentioned in the C1 to C6 alkyl group and the phenyl group in the above Ra and Rb, among which a methyl group is preferable.

The n in the formula (A) of the present invention is an integer of 1 to 10, and n=3 is preferable from viewpoints of low cost in an industrial scale and availability in a large amount.

The ophthalmic lens and the culture substrate material for cells and organs of the present invention are preferably obtained by polymerizing the cyclic siloxane compound of the formula (A) together with a hydrophilic monomer. The hydrophilic monomer is exemplified by an alcohol and a nitrogen-containing monomer, including specifically 2-hydroxyethyl methacrylate (2-HEMA), glycerol methacrylate (GMA), N-vinyl-2-pyrrolidone (NVP), N,N-dimethyl acrylamide (DMAA), 1-methyl-3-methylene-2-pyrolidinone (NMMP), etc. Those may be used singly or in combination of two or more thereof. Particularly a nitrogen-containing monomer is preferable from a viewpoint of suppressing turbidity of the resulting ophthalmic lens. DMAA is preferable for increasing transparency, and NVP is preferable from viewpoints of improvement of tackiness and a lipid-sticking property. The hydrophilic polymer is particularly preferably used in production of a silicone hydrogel contact lens.

In a case where the hydrophilic polymer is contained, a weight ratio of the cyclic siloxane compound shown by the formula (A) to the hydrophilic monomer is preferably 20 : 80 to 80 : 20. When the ratio of the cyclic siloxane compound shown by the formula (A) is less than 20 % by weight, the shape-holdability of the resulting ophthalmic lens is inferior and becomes flat, and thus wearing tends to be difficult. When the ratio of the cyclic siloxane compound shown by the formula (A) is over 80 % by weight, flexibility and repulsion of the produced lens tends to be lowered.

The cyclic siloxane compound shown by the formula (A) can be incorporated with a cross-linking agent.

The cross-linking agent is for giving a reinforcing effect, and exemplified by ethylene glycol dimethacrylate (EDMA), allyl methacrylate (AMA), diethylene glycol allyl ether (TRIAM), etc. Those may be used singly or in combination of two or more thereof. Among them, EDMA or a mixture of EDMA and AMA is preferably used as the cross-linking agent from a viewpoint of keeping suitable flexibility in the ophthalmic lens.

A content of the cross-linking agent is preferably 0.01 to 5 % by weight relative to the cyclic siloxane compound shown by the formula (A). When the content of the cross-linking agent is less than 0.01 % by weight, strength of the resulting ophthalmic lens becomes lower, and when over 5 % by weight, flexibility of the ophthalmic lens obtained tends to be lowered because of increased cross-linked structure ratio.

The ophthalmic lens, and the culture substrate material for cells and organs of the present invention are preferably produced by polymerizing the cyclic siloxane compound shown by the formula (A) together with a hydrophilic polymer and a cross-linking agent. In a case of co-using the hydrophilic polymer and the cross-linking agent, a content of the cross-linking agent is preferably 0.01 to 3 % by weight relative to the cyclic siloxane compound shown by the formula (A). When the content of the cross-linking agent is less than 0.01 % by weight, shape-holdability of the resulting ophthalmic lens and the culture substrate material tends to be inferior and become flat. Further, mounting of the ophthalmic lens tends to be difficult. When the content of the cross-linking agent is over 3 % by weight, though the shape-holdability becomes good, the lens becomes rigid (hard) and thus resulting ophthalmic lens and the culture substrate material tend to be fragile. Further, wearing of the ophthalmic lens tends to be difficult.

The method for polymerizing the cyclic siloxane compound shown by the formula (A) is not specifically restricted, and the polymerization can be conducted be a generally used polymerization method such as photo-polymerization and heat polymerization.

The ophthalmic lens of the present invention preferably has a Young's Modulus of 0.1 to 5 MPa, still preferably 0.2 to 1.1 MPa. When the Young's Modulus is less than 0.1 MPa, strength of the resulting ophthalmic lens tends to be lowered, and when it is over 5 MPa, on the other hand, the lens tends to be rigid and its wearing feeling tends to be inferior.

Further, the ophthalmic lens of the present invention preferably shows an oxygen-permeation coefficient (Dk) of $40 \times 10^{-11}$ $(cm^2/sec) \cdot (mLO_2/(mL \times mmHg))$ or more. When it is less than $40 \times 10^{-11}$ $(cm^2/sec) \cdot (mLO_2/(mL \times mmHg))$, the resultant tends to unsuitable to an ophthalmic lens.

A water content of the ophthalmic lens of the present invention is preferably not more than 90 %, still preferably 20 to 80 %. When it is over 90 %, strength of the resulting ophthalmic lens tends to be lowered.

The ophthalmic lens obtained in the present invention includes a contact lens, an intraocular lens, a corneal inlay, etc. The culture substrate material for cells and organs includes a substrate material used in production of a culture skin, a substrate material used as a skeleton of an artificial bone, etc. and the material is for seeding cells on or in the said substrate material. The container for living things means a general culture container used in cultivation of cells.

Still further, the present invention relates to a transparent gel obtained by polymerizing the cyclic siloxane compound shown by the formula (A) and a thermosetting polyimide silicone resin soluble in an organic solvent which contains structural units shown by the following formula (B-1) and (B-2).

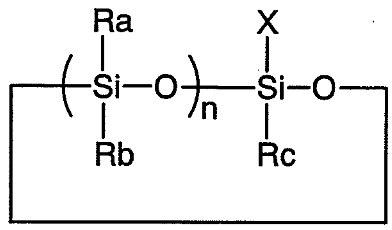

(A)

(wherein Ra or Rb is a hydrogen atom or a monovalent hydrocarbon group which may be substituted by a fluorine atom, and they are same or different with each other, Rc is a C1 to C6 alkyl group or a phenyl group, X is an organic group containing an unsaturated aliphatic bond, and n is an integer of 1 to 10).

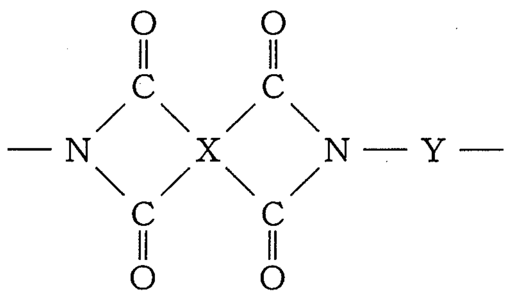

(B - 1)

[wherein X is a tetravalent organic group having 4 or more carbon atoms, providing that plural of -CO- groups are not bound to one carbon atom in X, and Y is a diamine residue shown by the general formula (1) or (2)

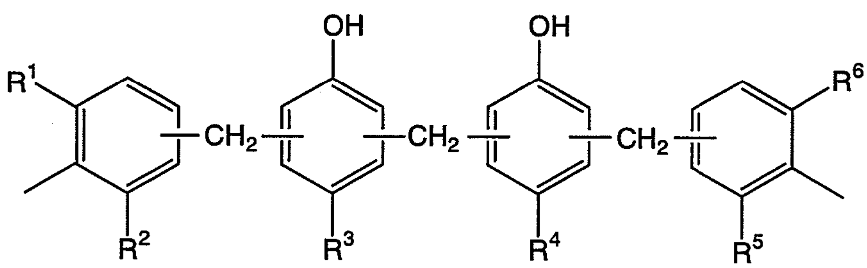

(1)

(wherein $R^1$ to $R^6$ are a hydrogen atom or a C1 to C6 alkyl group, which may be same or different)

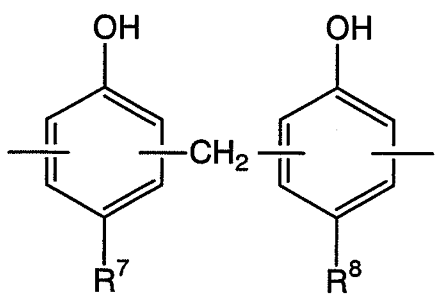

(2)

(wherein $R^7$ and $R^8$ are a hydrogen atom or a C1 to C6 alkyl group, which may be same or different)]

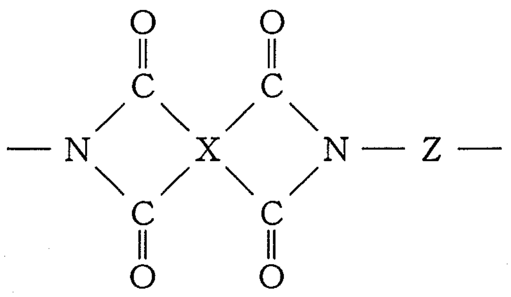

(B-2)

[wherein X is a tetravalent organic group having 4 or more carbon atoms, providing that plural of -CO- groups are not bound to one carbon atom in X, and Z is a diamine residue shown by the general formula (3)

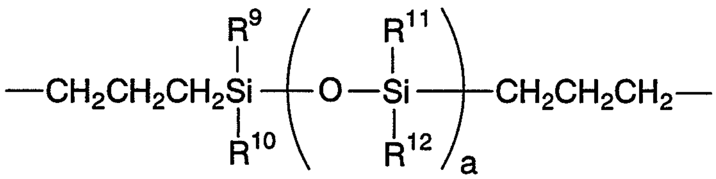

(3)

(wherein $R^9$ to $R^{12}$ are a C1 to C8 substituted or unsubstituted monovalent hydrocarbon group, which may be same or different, and a is an integer of 1 to 100)].

The cyclic siloxane compound shown by the formula (A) can be used within preferable fields of embodiments such as an ophthalmic lens, a culture substrate material for cells and organs and a container for living things.

The hydrophilic monomer is exemplified by a monomer used in an ophthalmic lens, a culture substrate for cells and organs, etc.

The transparent polyimide silicone resin is not specifically restricted so far as it contains the above structure (the transparent polyimide silicone resin disclosed in Japanese Patent Publication No. 2004-149777A), and one having the structure of the formula (6) is preferably used from viewpoints of transparency, flexibility and surface properties.

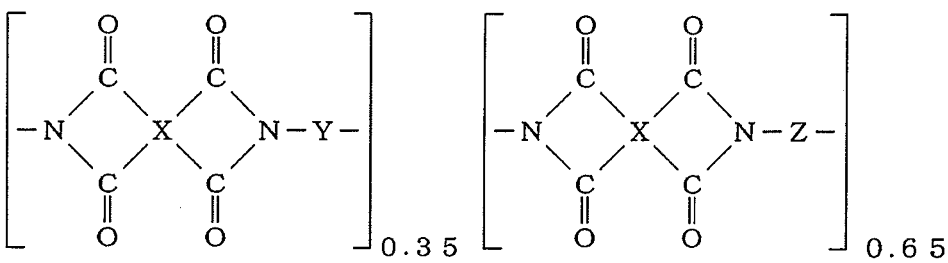

(6)

wherein X is

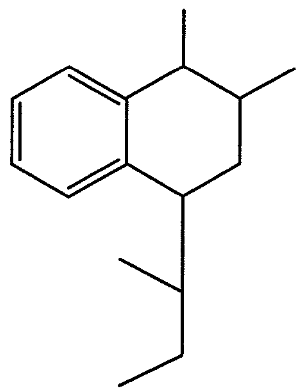

Y is

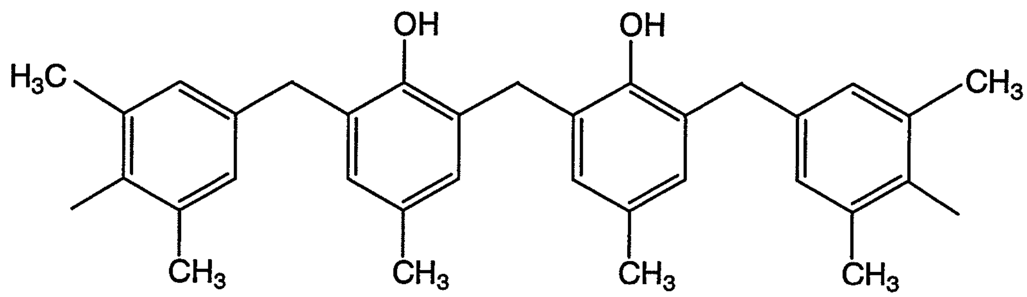

Z is

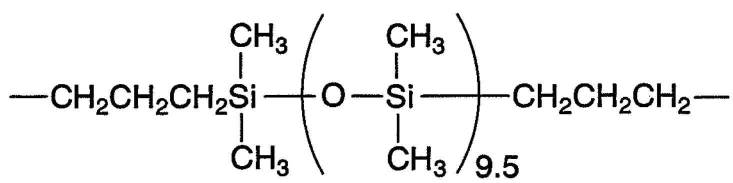

To a mixture of the cyclic siloxane compound shown by the formula (A), the hydrophilic monomer and the polyimide silicone resin may be added other ingredients including a silicone-containing monomer such as tris (trimethyl siloxy)silyl propyl methacrylate and tris (trimethyl siloxy)silyl styrene, a silicone-containing macromonomer such as polysiloxane macromonomer disclosed in International Patent Publication No. WO 01/071415, and the like.

The transparent gel of the present invention is preferably obtained by dissolving a polyimide silicone resin shown by the formula (B-1) or (B-2) into a mixture of the cyclic siloxane compound shown by the formula (A) and the hydrophilic monomer, followed by polymerizing.

A mixed ratio of the cyclic siloxane compound shown by the formula (A) to the hydrophilic monomer is preferably 90 : 10 to 10 : 90 by weight. When it is less than 10 % by weight, a mechanical strength of the resulting polymer tends to be lowered, and when it is over 90 % by weight, the resultant tends to be rigid and lowered flexible.

A content of the polyimide silicone resin is preferably not more than 50 parts by weight relative to the above mixture. When it is over 50 parts by weight, the resultant tends to be rigid.

The polymerization reaction is conducted by a conventional manner such as heat polymerization, photo polymerization and mold polymerization (e.g. resin mold). The polymerization is conducted appropriately with the addition of a conventional polymerization initiator such as a heat polymerization initiator and a photo polymerization initiator. A solution polymerization under addition of a solvent may also be conducted. The solvent is not specifically restricted, and

use can be made of THF, isopropyl alcohol, acetone, hexane, etc.

A polymerization temperature can be set forth according to the polymerization method, and in a case of heat polymerization, it is in a range of 60 to 120˚C, preferably 80 to 100˚C. In a case of polymerization at lower than 60˚C, no polymerization proceeds and non-polymerized monomer amount increases, and when over 120˚C, polymerization does not proceed, and an amount of non-polymerized monomer is increased, and also a mold tends to be deformed because of poor heat resistance, when a mold made of polypropylene (PP) is used.

The resulting transparent gel shows transparency and suitable flexibility, and thus it can be applied to an ophthalmic lens, a culture substrate for cells and organs, and a container for living things without surface treatment.

EXAMPLE

Mixed solutions were prepared according to Tables 2 to 7, and filled in a plate mold made of polypropylene, and UV polymerization was conducted for 30 minutes using a UV-ray curing machine (EYE GRAPHICS CO., LTD.) to give polymer samples. The samples were subjected to elution treatment with distilled water, and distilled water was replaced with saline, followed by sterilizing with an autoclave.

(Wettability, Stickiness and Tackiness)

Wettability, stickiness and tackiness of the plate surface of the resultant were evaluated by sensory test based upon feeling (wettability, stickiness, tackiness) after rubbing by fingers.

TABLE 1

| | ◎ | ○ | Δ | × |
|---|---|---|---|---|
| Wettability | very good | good | slightly inferior | inferior |
| Stickiness | completely none | | observed slightly | observed |
| Tackiness | completely none | | observed slightly | observed |

(Lipid adhesion)

After the resulting plates were immersed in an artificial lacrimal fluid (0.7 % aqueous lysozyme solution) for 16 hours or 40 hours, an amount of lipid adhered to the plates was analyzed using GC/MSD (5973N MSD system, Agilent Technologies).

(Refraction index Na-D)

A refraction index at 20˚C (Na-D line) of the resulting lenses was measured by an Abbe refractometer (1-T, Atago Co., Ltd.).

(Oxygen permeability (oxygen permeation index) Dk)

Dk of the resulting lenses were measured after an electrode method by an oxygen permeation measurement machine (Rikagaku Seiki Kogyo K.K.).

(Water content)

The plates were immersed in a saline for 16 hours to hydrate, and the surfaces were rightly wiped and the weights were measured (W1: g). Then the plates were placed in a drier at 105˚C for 16 hours, and then cooled to a room temperature in a desiccator, and weights of the dried plates were measured (W2: g). The water contents were calculated by the following equation:

$$(W1 - W2) / W1 \times 100 (\%)$$

(Young's Modulus)

Tensile test was conducted using a multi-role tester (Model 4301, Instron Japan Co., Ltd.), and an average value of each 5 plates was calculated to give Young's Modulus. Young's Modulus shows extension, and a large value means short extension and a small value means long extension.

(Stress Relaxation Rate)

Using a punch through tester, around 20 g load was given to the plates, and the stress after 30 and 60 seconds was measured, and the relaxation rate was calculated according the following equation compared with the initial load. An average value of each 3 plates was referred to the relaxation rate. The stress is an index of repulsion and as its value becomes large, the article becomes difficult to reform to the original form, and a small value means easy reform.

$$\text{Relaxation rate (\%)} = \{ \text{(initial load (g))} - \text{(stress after predetermined time (g))} \} / \text{initial load (g)} \times 100$$

TABLE 2

(Dimension of components: % by weight)

| | Ex. | | | | | | Com. Ex. | |
|---|---|---|---|---|---|---|---|---|
| | 1 | 2 | 3 | 4 | 5 | 6 | 1 | 2 |
| <u>Formula (A)</u> | | | | | | | | |
| S-502FCT | 50 | | 50 | 50 | | | | |
| S-502CT | | 50 | | | 50 | 50 | | |
| SK-5001 | | | | | | | 50 | 50 |
| <u>Hydrophilic monomer</u> | | | | | | | | |
| NVP | | | 50 | | 50 | | 50 | |
| DMAA | | | | 50 | | 50 | | 50 |
| <u>Cross-linking agent</u> | | | | | | | | |
| EDMA | 1 | 1 | 0.5 | 0.5 | 0.5 | 0.5 | 0.5 | 0.5 |
| <u>Photo polymerization initiator</u> | | | | | | | | |
| D1173 | 0.5 | 0.5 | 0.5 | 0.5 | 0.5 | 0.5 | 0.5 | 0.5 |
| <u>Physical properties</u> | | | | | | | | |
| Wettability | ○ | ○ | ◎ | ○ | ○ | ○ | × | × |
| Stickiness | ○ | ○ | ◎ | ◎ | ◎ | ◎ | × | × |
| Lipid-adhesion property (immersion for 16 hours) ($\mu g/cm^2$) | 1.26 | 1.30 | 0.13 | 0.77 | - | 1.11 | - | 4.61 |
| Lipid-adhesion property (immersion for 40 hours) ($\mu g/cm^2$) | - | - | 0.25 | 0.74 | - | 3.56 | - | 2.41 |
| Refractive index | 1.42 | 1.45 | 1.40 | 1.40 | - | 1.41 | - | 1.42 |
| Dk | 58 | 55 | 46 | 44 | - | 59 | - | 77 |
| Thickness of sample (mm) | 0.286 | 0.285 | 0.293 | 0.304 | - | 0.297 | - | 0.299 |
| Water content (%) | 0 | 0 | 45 | 52 | 47 | 53 | 51 1 | 50 |

-: not measured
Dk: $\times 10^{-11}$ ($cm^2$/sec) · ($mLO_2$/(mL $\times$ mmHg))

· S-502CT (3-methacryloxypropyl) heptamethyl cyclotetrasiloxane

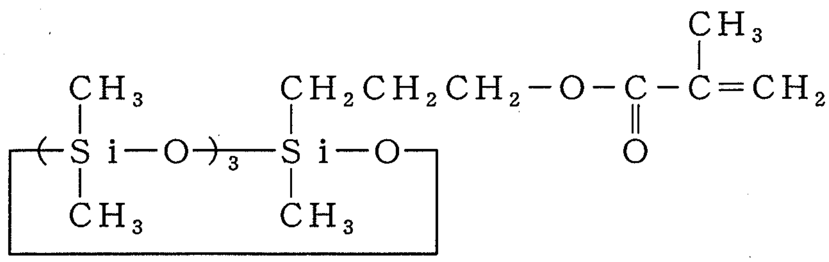

· S-502FCT (3-methacryloxypropyl)tris (3,3,3-trifluoropropyl)tetramethyl cyclotetrasiloxane

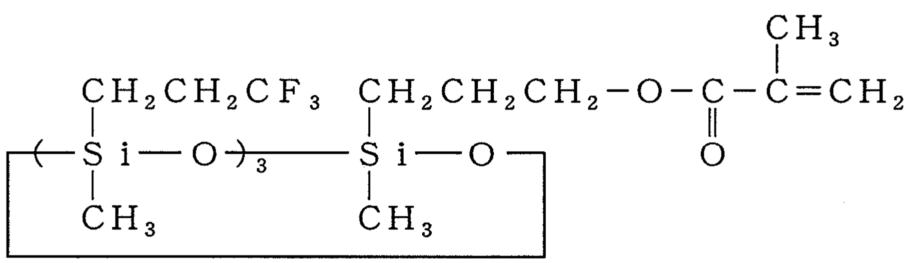

· SK-5001: tris(trimethyl siloxy)silyl propyl methacrylate (MW: 422.82, Shin-Etsu Chemical Co., Ltd.)
· NVP: N-vinyl-2-pyrrolidone
· DMAA: N,N-dimethylacrylamide
· NMMP: 1-methyl-3-methylene-2-pyrrolidinone
· EDMA: ethylene glycol dimethacrylate
· D1173: 2-hydroxy-2-methyl-1-phenyl-propan-1-on *(**Darocure*** (registered trade mark) 1173)

TABLE 3
(Dimension of components: % by weight)

| | Ex. | | | |
|---|---|---|---|---|
| | 7 | 8 | 9 | 10 |
| S-502FCT | 50 | 50 | 50 | 50 |
| S-502CT | | | | |
| SK-5001 | | | | |
| NVP | 45 | 40 | 35 | 25 |
| DMAA | 5 | 10 | 15 | 25 |
| EDMA | 0.5 | 0.5 | 0.5 | 0.5 |
| D1173 | 0.5 | 0.5 | 0.5 | 0.5 |
| <u>Physical properties</u> | | | | |
| Wettability | ◎ | ◎ | ◎ | ○ |
| Stickiness | ◎ | ◎ | ◎ | ◎ |
| Lipid-adhesion property (immersion for 16 hours) ($\mu g/cm^2$) | 0.53 | 0.86 | 0.80 | 0.73 |
| Lipid-adhesion property (immersion for 40 hours) | - | - | - | - |

(continued)

(Dimension of components: % by weight)

| | Ex. | | | |
|---|---|---|---|---|
| | 7 | 8 | 9 | 10 |
| (μg/cm$^2$) | | | | |
| Refractive index | - | - | - | - |
| Dk | - | - | - | - |
| Thickness of sample (mm) | - | - | - | - |
| Water content (%) | - | - | - | - |

-: not measured

Dk: $\times 10^{-11}$ ($cm^2$/sec) · ($mLO_2$/mL $\times$ mmHg))

TABLE 4

(Dimension of components: % by weight)

| | Ex. | | | | | | | | |
|---|---|---|---|---|---|---|---|---|---|
| | 11 | 12 | 13 | 14 | 15 | 16 | 17 | 18 | 19 |
| S-502FCT | 10 | 20 | 30 | 40 | 50 | 60 | 70 | 80 | 90 |
| NVP | 90 | 80 | 70 | 60 | 50 | 40 | 30 | 20 | 10 |
| EDMA | 0.5 | 0.5 | 0.5 | 0.5 | 0.5 | 0.5 | 0.5 | 0.5 | 0.5 |
| D1173 | 0.5 | 0.5 | 0.5 | 0.5 | 0.5 | 0.5 | 0.5 | 0.5 | 0.5 |
| Physical properties | | | | | | | | | |
| Wettability | ◎ | ◎ | ◎ | ◎ | ◎ | ◎ | ◎ | ◎ | ◎ |
| Stickiness | ◎ | ◎ | ◎ | ◎ | ◎ | ◎ | ◎ | ◎ | ◎ |
| Tackiness | ◎ | ◎ | ◎ | ◎ | ◎ | ◎ | ◎ | ◎ | ◎ |

TABLE 5

(Dimension of components: % by weight)

| | Ex. | | | | | | | | | | |
|---|---|---|---|---|---|---|---|---|---|---|---|
| | 20 | 21 | 22 | 23 | 24 | 25 | 26 | 27 | 28 | 29 | 30 |
| S-502FCT | 50 | 50 | 50 | 50 | 50 | 50 | 50 | 50 | 50 | 50 | 50 |
| NVP | 50 | 45 | 40 | 35 | 30 | 25 | 20 | 15 | 10 | 5 | 0 |
| DMAA | 0 | 5 | 10 | 15 | 20 | 25 | 30 | 35 | 40 | 45 | 50 |
| EDMA | 0.5 | 0.5 | 0.5 | 0.5 | 0.5 | 0.5 | 0.5 | 0.5 | 0.5 | 0.5 | 0.5 |
| D1173 | 0.5 | 0.5 | 0.5 | 0.5 | 0.5 | 0.5 | 0.5 | 0.5 | 0.5 | 0.5 | 0.5 |
| Physical properties | | | | | | | | | | | |
| Wettability | ◎ | ◎ | ◎ | ○ | ○ | ○ | ○ | Δ | Δ | Δ | Δ |
| Stickiness | ◎ | ◎ | ◎ | ◎ | ◎ | ◎ | ◎ | ◎ | ◎ | ◎ | ◎ |
| Tackiness | ◎ | ◎ | ◎ | ◎ | ◎ | ◎ | ◎ | ◎ | ◎ | ◎ | ◎ |
| Young's Modulus (MPa) | 4.961 | 1.047 | 0.427 | 0.320 | 0.394 | 0.346 | 0.305 | 0.268 | 0.197 | 0.136 | 0.192 |
| Stress relaxation rate after 30 seconds (%) | 38.5 | 28.6 | 22.5 | 20.7 | 19.6 | 22.5 | 23.2 | 23.1 | 23.9 | 25.7 | 23.7 |

(continued)

(Dimension of components: % by weight)

| | Ex. | | | | | | | | | | |
|---|---|---|---|---|---|---|---|---|---|---|---|
| | 20 | 21 | 22 | 23 | 24 | 25 | 26 | 27 | 28 | 29 | 30 |
| Stress relaxation rate after 60 seconds (%) | 41.9 | 31.6 | 26.3 | 24.5 | 23.3 | 25.9 | 27.3 | 27.6 | 28.1 | 30.1 | 28.1 |

TABLE 6

(Dimension of components: % by weight)

| | Ex. | | | | | | |
|---|---|---|---|---|---|---|---|
| | 31 | 32 | 33 | 34 | 35 | 36 | 37 |
| S-502FCT | 50 | 50 | 50 | 50 | 50 | 50 | 50 |
| NMMP | 0 | 0 | 0 | 0 | 30 | 20 | 10 |
| NVP | 30 | 20 | 10 | 0 | 0 | 0 | 0 |
| DMAA | 20 | 30 | 40 | 50 | 20 | 30 | 40 |
| EDMA | 1 | 1 | 1 | 1 | 1 | 1 | 1 |
| D1173 | 0.5 | 0.5 | 0.5 | 0.5 | 0.5 | 0.5 | 0.5 |
| Physical properties | | | | | | | |
| Wettability | ○ | ○ | Δ | Δ | Δ | Δ | Δ |
| Stickiness | ◎ | ◎ | ◎ | ◎ | ◎ | ◎ | ◎ |
| Tackiness | ◎ | ◎ | ◎ | ◎ | ◎ | ◎ | ◎ |
| Young's Modulus (MPa) | 0.516 | 0.415 | 0.446 | 0.418 | 0.463 | 0.559 | 0.380 |
| Stress relaxation rate after 30 seconds (%) | 22.3 | 20.9 | 20.8 | 22.2 | 24.1 | 23.5 | 25.1 |
| Stress relaxation rate after 60 seconds (%) | 24.4 | 23.3 | 24.6 | 26.2 | 29.0 | 26.4 | 007 |

TABLE 7

(Dimension of components: % by weight)

| | Ex. | | | | | | | | | |
|---|---|---|---|---|---|---|---|---|---|---|
| | 38 | 39 | 40 | 41 | 42 | 43 | 44 | 45 | 46 | 47 |
| S-502FCT | 50 | 50 | 50 | 50 | 50 | 50 | 50 | 50 | 50 | 50 |
| NVP | 40 | 30 | 20 | 10 | 40 | 30 | 20 | 10 | 0 | 0 |
| DMAA | 10 | 20 | 30 | 40 | 10 | 20 | 30 | 40 | 50 | 50 |
| EDMA | 0.25 | 0.25 | 0.25 | 0.25 | 0.75 | 0.75 | 0.75 | 0.75 | 0.75 | 1 |
| D1173 | 0.5 | 0.5 | 0.5 | 0.5 | 0.5 | 0.5 | 0.5 | 0.5 | 0.5 | 0.5 |
| Physical properties | | | | | | | | | | |
| Wettability | ◎ | ◎ | ○ | Δ | ◎ | ○ | Δ | Δ | Δ | Δ |
| Stickiness | ◎ | ◎ | ◎ | ◎ | ◎ | ◎ | ◎ | ◎ | ◎ | ◎ |
| Tackiness | ◎ | ◎ | ◎ | ◎ | ◎ | ◎ | ◎ | ◎ | ◎ | ◎ |
| Young's Modulus (MPa) | 0.289 | 0.280 | 0.331 | 0.278 | 0.444 | 0.385 | 0.334 | 0.371 | 0.334 | 0.441 |
| Stress relaxation rate after 30 seconds (%) | 25.4 | 21.3 | 22.0 | 23.0 | 20.1 | 20.2 | 22.8 | 23.6 | 23.0 | 23.3 |
| Stress relaxation rate after 60 seconds (%) | 30.2 | 25.1 | 25.5 | 27.1 | 23.8 | 23.2 | 26.6 | 27.2 | 27.0 | 26.5 |

As result from Comparative Examples 1 and 2 of Table 2, when a conventional silicone-containing monomer was used, surface wettability was inferior and tackiness was observed even using with a hydrophilic monomer. On the other hand, when the cyclo siloxane compound of the present invention shown by the formula (A) was used, surface wettability was good and plates having no tackiness were obtained even without using a hydrophilic monomer. Further, it was result from Examples 3 to 6, tackiness was improved by co-using a hydrophilic monomer.

Also regarding the lipid adhesion amount, as result from Comparative Example 2, the amount was large when a conventional silicone-containing monomer was used, while the amount was considerably reduced when the cyclic siloxane compound of the present invention shown by the formula (A) was used.

Among the Examples, one wherein surface wettability was good, no tackiness was observed and lipid was difficult to adhere was Example 3 wherein a hydrophilic monomer was co-used.

From the evaluation of the refractive index, the oxygen-permeation index, the water content, the Young's Modulus and the stress relaxation rate, they were understood to be within a range sufficiently capable of applying to a contact lens.

INDUSTRIAL APPLICABILITY

According to the present invention, an ophthalmic lens, a culture substrate material for cells and organs and a container for living things wherein tackiness and stickiness are reduced, lipid adhesion ability is excellent and surface wettability is also excellent can be provided.

**Claims**

1. An ophthalmic lens, which is obtained by polymerizing a cyclic siloxane compound shown by the following formula (A)

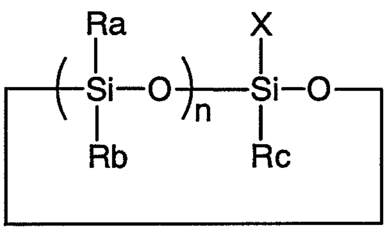

(A)

(wherein Ra and Rb are, same or different, a hydrogen atom or a monovalent hydrocarbon group which may be substituted by a fluorine atom, Rc is a C1 to C6 alkyl group or a phenyl group, X is an organic group containing an aliphatic unsaturated bond, and n is an integer of 1 to 10).

2. The ophthalmic lens of Claim 1, which is one obtained by polymerizing a hydrophilic monomer together with a cyclic siloxane compound.

3. A culture substrate material for cells or organs, which is obtained by polymerizing a cyclic siloxane compound shown by the formula (A)

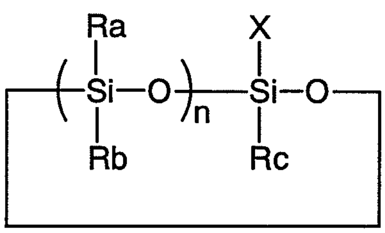

(A)

(wherein Ra and Rb are, same or different, a hydrogen atom or a monovalent hydrocarbon group which may be

substituted by a fluorine atom, Rc is a C1 to C6 alkyl group or a phenyl group, X is an organic group containing an aliphatic unsaturated bond, and n is an integer of 1 to 10).

**4.** The culture substrate material for cells or organs of Claim 3, which is obtained by polymerizing a hydrophilic monomer together with a cyclic siloxane compound.

**5.** A container for living things, which is obtained by polymerizing a cyclic siloxane compound shown by the formula (A)

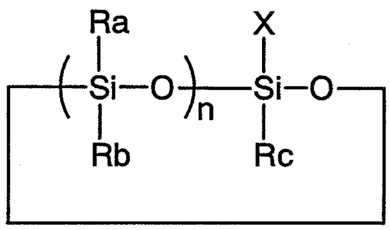

(A)

(wherein Ra and Rb are, same or different, a hydrogen atom or a monovalent hydrocarbon group which may be substituted by a fluorine atom, Rc is a C 1 to C6 alkyl group or a phenyl group, X is an organic group containing an aliphatic unsaturated bond, and n is an integer of 1 to 10).

**6.** A transparent gel, which is obtained by polymerizing a cyclic siloxane compound shown by the following formula (A), a hydrophilic monomer and a thermosetting polyimide silicone resin soluble in an organic solvent, which contains structural units shown by the following formula (B-1) and (B-2)

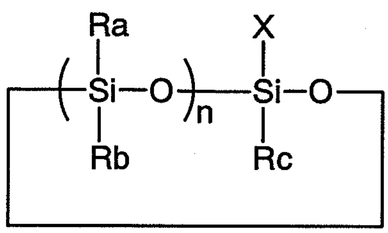

(A)

(wherein Ra or Rb is a hydrogen atom or a monovalent hydrocarbon group which may be substituted by a fluorine atom, and they are same or different with each other, Rc is a C1 to C6 alkyl group or a phenyl group, X is an organic group containing an unsaturated aliphatic bond, and n is an integer of 1 to 10).

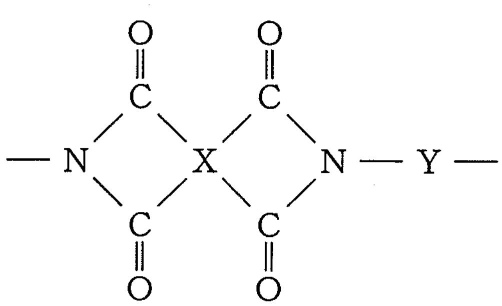

(B－1)

[wherein X is a tetravalent organic group having 4 or more carbon atoms, providing that plural of -CO- groups are not bound to one carbon atom in X, and Y is a diamine residue shown by the general formula (1) or (2)

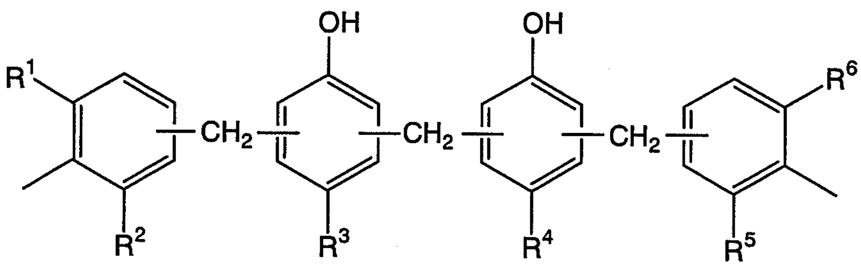

(1)

(wherein each of $R^1$ to $R^6$ is, same or different, a hydrogen atom or a C 1 to C6 alkyl group).

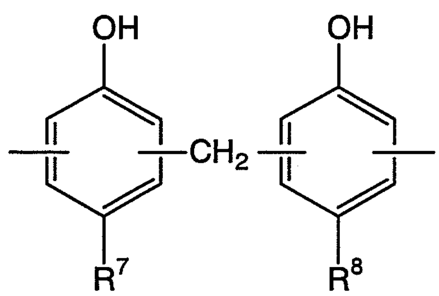

(2)

(wherein $R^7$ and $R^8$ are, same or different, a hydrogen atom or a C 1 to C6 alkyl group)]

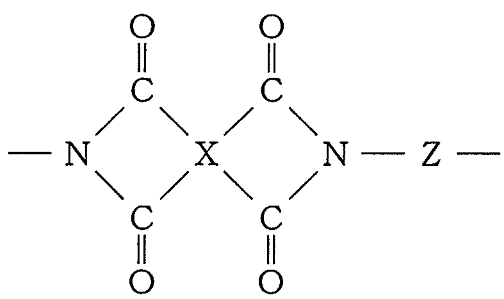

(B-2)

[wherein X is a tetravalent organic group having 4 or more carbon atoms, providing that plural of -CO- groups are not bound to one carbon atom in X, and Z is a diamine residue shown by the general formula (3)

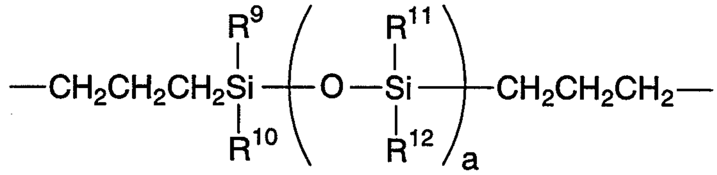

(3)

(wherein $R^9$ to $R^{12}$ are, same or different, a substituted or unsubstituted monovalent hydrocarbon group of C1 to C8, and a is an integer of 1 to 100)].

**7.** A method for producing the transparent gel of Claim 6 comprising dissolving a polyimide silicone resin containing structural units shown by the formula (B-1) and (B-2) in a mixture of a cyclic siloxane compound shown by the formula (A) and a hydrophilic monomer and polymerizing the same.

## INTERNATIONAL SEARCH REPORT

International application No.
PCT/JP2006/311275

A. CLASSIFICATION OF SUBJECT MATTER
*A61L27/00*(2006.01)i, *C12M3/00*(2006.01)i, *G02C7/04*(2006.01)i, *C08F30/08*
(2006.01)n

According to International Patent Classification (IPC) or to both national classification and IPC

B. FIELDS SEARCHED

Minimum documentation searched (classification system followed by classification symbols)
A61L27/00, C12M3/00, G02C7/04, C08F30/08

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 04-154825 A (JAPAN SYNTHETIC RUBBER CO., LTD.),<br>27 May, 1992 (27.05.92),<br>Claims; examples; page 10, upper right column, line 9 to lower left column, line 17<br>& JP 3043390 B2 | 1,2<br>3-7 |
| X<br>Y | JP 61-057612 A (NIPPON CONTACT LENS),<br>24 March, 1986 (24.03.86),<br>Examples 27, 28, 37, 57, 58; page 4, lower right column, line 19 to page 5, upper left column, the last line<br>& US 4602074 A & JP 93015724 B | 1,2<br>3-7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 31 July, 2006 (31.07.06) | 08 August, 2006 (08.08.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2006/311275

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 60-165617 A (TOKYO CONTACT LENS CO., LTD.),<br>28 August, 1985 (28.08.85),<br>Claims; example 7<br>& JP 89020406 B | 1,2<br>3-7 |
| X<br>Y | JP 62-054220 A (NIPPON CONTACT LENS),<br>09 March, 1987 (09.03.87),<br>Examples 22, 23<br>& US 4743667 A & JP 89020408 B | 1,2<br>3-7 |
| Y | JP 11-302409 A (TORAY IND INC.),<br>02 November, 1999 (02.11.99),<br>Full text<br>(Family: none) | 3-5 |
| Y | JP 2004-149777 A (SHINETSU CHEM CO., LTD.),<br>27 May, 2004 (27.05.04),<br>Full text<br>& EP 1408067 A1 & US 2004/072982 A1<br>& EP 1408067 B1 | 6,7 |
| A | JP 07-149902 A (SHINETSU CHEM IND CO., LTD.),<br>13 June, 1995 (13.06.95),<br>& JP 2856378 B2 | 1-7 |
| A | JP 08-245790 A (ASAHI KASEI KOGYO KABUSHIKI KAISHA),<br>24 September, 1996 (24.09.96),<br>& JP 3406112 B2 | 1-7 |

Form PCT/ISA/210 (continuation of second sheet) (April 2005)

**INTERNATIONAL SEARCH REPORT**

International application No.
PCT/JP2006/311275

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

Claims 1-7 of the present international application are considered to relate to the groups of inventions A to C, as follows.

A. The inventions of claims 1 and 2 relate to an ophthalmic lens produced by polymerizing a cyclic siloxane compound of the formula (A).

B. The inventions of claims 3-5 relate to a cell or organ culture substrate and a container for a biological material produced by polymerizing a cyclic siloxane compound of the formula (A).

C. The inventions of claims 6 and 7 relate to a transparent gel produced by polymerizing a cyclic siloxane of the formula (A), a hydrophilic

(continued to extra sheet)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

**INTERNATIONAL SEARCH REPORT**

International application No.
PCT/JP2006/311275

Continuation of Box No.III of continuation of first sheet(2)

monomer and a polyimide silicone resin.

The technical matter common to these three groups of inventions is a polymerized product of a cyclic siloxane compound of the formula (A). However, this technical matter is already known before the filing of the present international application (for example, see JP 04-154825 A). Consequently, the polymerized product of the cyclic siloxane compound is included within the scope of the prior art, and therefore the common matter (i.e., the polymerized product of the cyclic siloxane compound) is not a special technical matter in the meaning within PCT Rule 13.2, second sentence.

Such being the case, the groups of inventions A to C cannot be considered as a group of inventions so linked as to form a single general inventive concept, and therefore do not comply with the requirement of unity of invention.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 1256537 A
- JP 7149902 A
- JP 2004149777 A
- WO 01071415 A